# EUROPEAN PATENT APPLICATION

(11) **EP 3 608 395 A1**
(43) Date of publication of application: **12.02.2020**
(21) Application number: 18188495.8
(22) Date of filing: 10.08.2018
(51) Int. Cl.: C12N 1/00, C12P 17/18, C12P 25/00, C12P 39/00

(54) **DIRECTED EVOLUTION OF A MUTUALISTIC COMMUNITY OF ORGANISMS FOR THE PRODUCTION OF A NUTRIENT OR A NUTRACEUTICAL**

(71) Applicant: European Molecular Biology Laboratory, 69117 Heidelberg (DE)
(72) Inventor: KONSTANTINIDIS, Dimitrios, 69117 Heidelberg (DE); PATIL, Kiran Raosaheb, 69120 Heidelberg (DE); PEREIRA, Filipa, 69120 Heidelberg (DE); MOUTINHO LUZ MACHADO, Carlos Daniel, 69115 Heidelberg (DE); KIM, Yongkyu, 69115 Heidelberg (DE)
(74) Representative: Winter, Brandl, Fürniss, Hübner, Röss, Kaiser, Polte - Partnerschaft mbB

(57) **Abstract**

The present invention relates to a method of production of a nutrient or a nutraceutical compound, a method for the generation of an improved nutrient-producing or nutraceutical compound-producing organism or community of organisms, and nutrient-producing or nutraceutical compound-producing organisms or communities of organisms.

## Description

### Technical field

The present invention relates to a method of production of a nutrient or a nutraceutical compound, a method for the generation of an improved nutrient-producing or nutraceutical compound-producing organism or community of organisms, and to nutrient-producing or nutraceutical compound-producing organisms or communities of organisms.

### Background of the Invention

Microbial communities as well as directed evolution harbor immense potential for biotechnological applications. Interest in understanding how microbial species are able to adapt and evolve in different environments is high, yet most of the times only single species are studied. Even though such approaches based on single species are useful, in reality the majority of species co-exist with others, forming communities.

For this reason, experimental studies on the community level are performed more and more often. Generally, in this type of studies strains are engineered to form a dependent, e.g. mutualistic, community and changes at their general growth performance is evaluated.

At the same time, numerous biotechnological applications have been achieved with the use of microbial communities wherein various organisms cooperate with each other, such as production of biopolymers and the production of biogas in anaerobic bioreactors. The reason for the success of such a cooperation is that the biosynthesis of compounds requires the activation of complex metabolic pathways, which most of the times are not complete in one species and cannot be engineered easily. Use of communities however enables division of labor between the members and enhances robustness (Hays SG et al.: "Better together: engineering and application of microbial symbioses"; Curr Opin Biotechnol. 2015 Dec; 36:40-9.).

Nutrients or nutraceutical compounds are exemplary compounds which are produced in such complex biosynthetic pathways. Nutraceuticals are food compounds that confer health benefits upon consumption, such as vitamins. Using nutrient- or nutraceutical compound-producing organisms or communities of organisms, it could be possible to produce these compounds using biosynthetic pathways instead of chemical synthesis processes and, additionally, increase the amounts of such useful compounds in certain foods in a targeted fashion.

This is particularly important given the numerous deficiencies of certain nutrients and nutraceutical compounds in developing countries as well as industrialized societies (LeBlanc et al.: "B-Group vitamin production by lactic acid bacteria - current knowledge and potential applications"; J Appl Microbiol. 2011 Dec; 111(6):1297-309.).

Thus, it is an object of the present invention to provide useful and advantageous methods for the preparation of nutrients and nutraceutical compounds such as vitamins using biosynthetic pathways in living organisms. Furthermore, it is an object of the present invention to provide methods for obtaining organisms with very high and increased ability of producing nutrients and nutraceutical compounds. Last, organisms and communities of organisms are sought which produce nutrients and nutraceutical compounds in a desirable and advantageous fashion.

### Summary of the Invention

These objects have been solved by the aspects of the present invention as specified hereinafter.

According to the first aspect of the present invention, a method for the production of a nutrient or a nutraceutical compound is provided comprising the provision of a parental mutualistic community of organisms comprising a first organism which is able to produce and excrete into the environment a first set of compounds and which requires said nutrient or nutraceutical compound to be produced for optimal proliferation, and a second organism which is able to produce and excrete into the environment said nutrient or nutraceutical compound, and which requires said first set of compounds for optimal proliferation, followed by cultivation of said parental mutualistic community of organisms in a culture comprising a growth medium which is devoid of or contains levels of the nutrient or nutraceutical compound and of the set of compounds to be excreted by said first organism which are insufficient to allow optimal proliferation of the first and the second organisms, and then harvesting the nutrient or nutraceutical compound from the culture medium after cultivation.

According to a preferred embodiment of the first aspect of the present invention, the first organism is a microbial strain and/or the second organism is a microbial strain.

According to another preferred embodiment of the first aspect of the present invention, the first organism is from the phylum *Ascomycota,* preferably from the class *Saccharomycetes,* more preferably from the genus *Saccharomyces,* particularly preferably of the species *Saccharomyces cerevisiae.*

According to yet another preferred embodiment of the first aspect of the present invention, the first organism is auxotrophic for the nutrient or nutraceutical compound to be produced.

According to a preferred embodiment of the first aspect of the present invention, the auxotrophy is due to functional impairment of a gene, preferably due to gene deletion, more preferably due to deletion of genes in the synthetic pathway for production of the nutrient or nutraceutical compound by the first organism.

According to a preferred embodiment of the first aspect of the present invention, the first set of compounds comprises amino acids, preferably the first set of compounds comprises amino acids glutamine and serine, more preferably the first set of compounds comprises the amino acids mentioned in Table 1 herein, particularly preferably the first set of compounds consists of amino acids glutamine and serine, alternatively preferably the first set of compounds consists of the amino acids mentioned in Table 1 herein.

According to another preferred embodiment of the first aspect of the present invention, the second organism is from the domain *Bacteria,* preferably from the group of lactic acid bacteria (LAB), more preferably of the species *Lactococcus lactis, Lactobacillus parakefiri* or *Brevibacterium casei.*

According to another preferred embodiment of the first aspect of the present invention, the second organism is auxotrophic for the first set of compounds, preferably the second organism is auxotrophic for one or more amino acids, more preferably the second organism is auxotrophic for one or more of the amino acids mentioned in Table 1 herein, particularly preferably the second organism is auxotrophic for glutamine and/or serine.

According to yet another preferred embodiment of the first aspect of the present invention, the second organism is a natural isolate and/or the second organism is not a genetically modified organism (GMO).

According to a preferred embodiment of the first aspect of the present invention, the nutrient or nutraceutical compound is a vitamin, preferably the nutrient or nutraceutical compound is a B vitamin, more preferably the nutrient or nutraceutical compound is Vitamin B2 (riboflavin) or Vitamin B9 (folate).

According to another preferred embodiment of the first aspect of the present invention, the growth medium does not comprise amino acids, in particular serine and glutamine, in levels sufficient to allow optimal proliferation of the first and second organisms, alternatively the growth medium does not comprise the nutrient or nutraceutical compound to be produced in levels sufficient to allow optimal proliferation of the first and second organisms, more preferably the growth medium does not comprise any of serine, glutamine or the nutrient or nutraceutical compound to be produced in levels sufficient to allow optimal proliferation of the first and second organisms, most preferably the growth medium does not comprise any of serine, glutamine, and Vitamin B2 or B9 in levels sufficient to allow optimal proliferation of the first and second organisms.

Preferably, a growth medium in accordance with the medium described in the Example section can be used. More preferably, a growth medium is used which does not comprise the compounds for which an auxotrophy exists in one of the organisms comprised in the mutualistic community of organisms.

According to the second aspect of the present invention, a method is provided for the generation of an improved nutrient-producing or nutraceutical compound-producing organism or community of organisms comprising cultivation of a parental mutualistic community of organisms as defined under the first aspect of the invention in one or multiple batches, determination of the nutrient-producing or nutraceutical compound-producing ability of each community and/or each batch after cultivation, followed by selection of a community of organisms which produces higher amounts of the nutrient or nutraceutical compound than the parental mutualistic community of organisms.

According to a preferred embodiment of the second aspect of the present invention, the nutrient-producing or nutraceutical compound-producing organism is subsequently isolated from the community of organisms selected in the step of selection of a community of organisms according to the method of the second aspect of the invention, preferably wherein the nutrient-producing or nutraceutical compound-producing organism is then propagated in monoculture.

According to another preferred embodiment of the second aspect of the present invention, the cultivation is carried out for at least a time which is equivalent to two average generation times of the slower growing first or second organism, preferably the cultivation is carried out for at least a time which is equivalent to ten average generation times of the slower growing first or second organism, more preferably twenty generation times, even more preferably fifty generation times.

According to a preferred embodiment of the second aspect of the present invention, the cultivation comprises serial transfers of the cultivated communities, preferably the cultivation comprises at least 3, more preferably 5, even more preferably ten, most preferably 15 serial transfers of the cultivated communities.

According to another preferred embodiment of the second aspect of the present invention, the nutrient-producing ability or nutraceutical compound-producing ability is determined by measuring the amount of nutrient or nutraceutical compound present in the medium, preferably the nutrient-producing ability or nutraceutical compound-producing ability is determined by analyzing the culture supernatant through a quantitative measurement of the nutrient or nutraceutical compound, more preferably the nutrient-producing ability or nutraceutical compound-producing ability is determined by analyzing culture supernatant samples using UPLC, HPLC, LC, colorimetric assays, fluorescence/spetcroscopic measurement, GC-MS, LC-MS, MS, enzyme assays or biological assays, most preferably the nutrient-producing ability or nutraceutical compound-producing ability is determined by analyzing culture supernatant samples using Ultra Performance Liquid Chromatography (UPLC).

According to yet another preferred embodiment of the second aspect of the present invention, the organisms selected for producing high amounts of the nutrient or nutraceutical compound are repeatedly subjected to the steps of the method as defined according to the second aspect of the present invention.

According to the third aspect of the present invention, a nutrient-producing or nutraceutical compound-producing community of organisms is provided as defined under the first aspect of the invention.

According to the fourth aspect of the present invention, a nutrient-producing or nutraceutical compound-producing organism obtained by the method of the second aspect of the present invention is provided.

### Description of Figures

Figure 1 shows (A) a graphical representation of auxotrophic yeast strains created with single deletions in the pathway for riboflavin production (modified from Gudipati et al. (2014) "The flavoproteome of the yeast Saccharomyces cerevisiae." Biochim Biophys Acta, 1844, 535-544.) and (B) the growth curve of the strain Δ*rib4* grown in rich medium and supplemented with different concentrations of riboflavin.
Figure 2 shows the growth pattern of the evolving populations of (A) *L. lactis* and (B) B. *casei* over a period of two months.
Figure 3 shows the determination of riboflavin concentration from supernatant monocultures of isolates of (A) *L. lactis* and (B) B. *casei* by measuring fluorescence at 440/520 nm with the parental strains shown first from the left.
Figure 4 shows (A) growth curves of three different *L. lactis* isolates and their parental strain in CDM35R- and (B) production levels of riboflavin and FMN as determined by UPLC analysis.
Figure 5 shows the determination of riboflavin concentration from supernatant monocultures of isolates after the second round of evolution of (A) *L. lactis* and (B) *B*. *casei* by measuring fluorescence at 440/520 nm with the parental strains shown first from the left.
Figure 6 shows (A) growth curves of five different *L. lactis* isolates and their parental strain in CDM35R-, (B) riboflavin levels produced by different strains through different time points, and (C) maximum production levels of riboflavin as determined by UPLC analysis.
Figure 7 shows (A) growth curves of five different *B*. *casei* isolates and their parental strain in CDM35R- and (B) production levels of riboflavin as determined by UPLC analysis.
Figure 8 shows (A) growth curves of the parental and two evolved *L. lactis* strains obtained after the first round of evolution in conditioned medium of wild type yeast cells, (B) growth curves of the parental and two evolved *L. lactis* strains obtained after the first round of evolution in two times diluted CDM35R-, and (C) growth curves of auxotrophic yeast cells in conditioned medium collected from the parental and two evolved *L. lactis* strains.

### Detailed Description of the Invention

The interplay and mutual support in communities of organisms of different species in nature as well as *in vitro* are subject to ongoing analysis and research. However, a detailed understanding of successfully establishing a functional proliferative community in a planned and defined manner exhibiting the desired and sought-after properties is still lacking.

Directed evolution of cultured organisms has also been tried and analyzed continuously to find a way for applying a driving force on such organisms towards a defined goal, such as obtaining organisms with increased toxin resistance, improved growth characteristics, or superior production of desired enzymes or nutrients.

Until now, a combination of these two applications for microbial communities has not been described. In this study, the inventors were successful to establish a mutualistic community, which is able to produce high yields of complex metabolic traits. Even more, the inventors were successful in providing a method using adaptive evolution to obtain communities and particular organisms which are able to produce even higher yields of such complex metabolic traits than what would be found in the starting strains taken from nature as natural isolates.

One of the aims of the inventors was the creation of strains with the desired phenotype, originating from a suitable environment, for example a dairy product such as kefir which can reach consumers. Thus, the possibility of creating non-GMO strains with improved characteristics and which may reach the consumer without any concern is the main advantage of this method. The compounds produced will be particularly useful for applications where engineered and GMO strains cannot be used, such as production of nutraceuticals.

The number of possible combinations of first and second organisms in theory is large and limited only by the requirement that one species (a natural variant or GMO if permitted) produces a desired compound that is required by the other species, which in turn produces something useful for the first species. As long as such combination can be made, e.g. by choosing/screening for natural variants or genetic engineering combined with suitable environment, the method can be applied.

Thus, according to the present invention, a method for the production of a nutrient or a nutraceutical compound is provided comprising the provision of a parental mutualistic community of organisms comprising a first organism which is able to produce and excrete into the environment a first set of compounds and which requires said nutrient or nutraceutical compound to be produced for optimal proliferation, and a second organism which is able to produce and excrete into the environment said nutrient or nutraceutical compound, and which requires said first set of compounds for optimal proliferation, followed by cultivation of said parental mutualistic community of organisms in a culture comprising a growth medium which is devoid of or contains levels of the nutrient or nutraceutical compound and of the set of compounds to be excreted by said first organism which are insufficient to allow optimal proliferation of the first and the second organisms, and then harvesting the nutrient or nutraceutical compound from the culture medium after cultivation.

The nutrient or nutraceutical compound to be produced by the methods of the present invention is preferably a vitamin, preferably a B vitamin, more preferably Vitamin B2 (riboflavin) or Vitamin B9 (folate). However, the present invention is not limited and it will be readily apparent to the skilled person that other nutrient or nutraceutical compounds may also be produced in an improved manner by using the methods of the present invention.

The parental mutualistic community of organisms is preferably made up of at least two different species, wherein one produces a desired compound that is required by the other, and wherein the other produces something useful for the first species. The organisms selected for the mutualistic community may be from the same species or from different species, preferably the organisms are from different species.

The first organism is able to produce and excrete into the environment a first set of compounds which are essential for the second organism. Preferably, the first set of compounds comprises building blocks used by the second organism, more preferably polymers or monomers derived from proteins, nucleic acids or poly- and oligosaccharides, such as amino acids, sugars or nucleic acids.

In addition, the first organism requires the nutrient or nutraceutical compound to be produced by the second organism for optimal growth and/or proliferation. In one preferred embodiment, the first organism has an auxotrophy for said nutrient or nutraceutical compound. Optimal growth and/or proliferation within the meaning of the present invention is understood as a growth and/or proliferation rate which could be reached by said organism in full medium and optimal growth conditions.

On the other hand, the second organism is able to produce and excrete into the environment the nutrient or nutraceutical compound which is to be produced by the methods of the present invention. In addition, the second organism requires the first set of compounds to be produced by the first organism for optimal growth and/or proliferation. In one preferred embodiment, the second organism has an auxotrophy for said first set of compounds.

According to a preferred embodiment, the auxotrophy of any of the strains of the present invention may be an inherent property of an organism obtained from a natural isolate. According to another preferred embodiment, the auxotrophy of the first or second organism may be caused by a functional impairment of a gene, preferably due to gene deletion, more preferably due to deletion of one or more genes in the synthetic pathway for the production of the nutrient or nutraceutical compound to be produced by the first organism. Preferably, functional impairment of a gene may be understood as a mutation of one or more nucleotides of said gene.

According to a preferred embodiment of the present invention, the first organism and/or the second organism are microbial organisms. More preferably, the organisms present in the mutualistic community may be selected from microbial organisms which can be cultivated in a laboratory or production facility, even more preferably in liquid culture. According to a particularly preferred embodiment of the methods of the present invention, the cultivation step is not carried out in a natural habitat of the organisms used therein. Preferably, the cultivation step is carried out in a laboratory or production facility, more preferably in liquid culture. According to a preferred embodiment of the invention, microbial organisms should be understood to include all unicellular organisms, more preferably the microbial organisms are to be selected from bacteria, protozoa, fungi and parasites, most preferably from bacteria and fungi.

Further, a culture is used for the cultivation step comprising a growth medium being either devoid of the nutrient or nutraceutical compound as well as of the first set of compounds or contains the nutrient or nutraceutical compound and the first set of compounds in levels which are insufficient to allow optimal proliferation of the first and second organisms. In this regard, levels which are insufficient to allow optimal proliferation are compared to proliferation in full medium at optimal growth conditions.

The step of harvesting the nutrient or nutraceutical compound from the culture medium is done as is commonly known for each respective nutrient or nutraceutical compound. Such methods are commonly known in the art as evidenced by the general knowledge document presenting such methods for the examples of water-soluble vitamins (cf. Ball G.F.M. (1994) Extraction of the water-soluble vitamins. In: Water-soluble Vitamin Assays in Human Nutrition. Springer, Boston, MA).

According to one preferred embodiment, the first and/or the second organism is derived from a natural isolate. In particular, it is preferred that the first and/or the second organism is not a genetically modified organism, more preferably the organism producing and excreting the nutrient or nutraceutical compound is not a genetically modified organism, particularly preferably the second organism is not a genetically modified organism.

According to the present invention, another method is provided for the generation of an improved nutrient-producing or nutraceutical compound-producing organism or community of organisms comprising cultivation of a parental mutualistic community of organisms as defined under the first aspect of the invention in multiple parallel batches, determination of the nutrient-producing or nutraceutical compound-producing ability of each community and/or each batch after cultivation, followed by selection of a community of organisms which produces higher amounts of the nutrient or nutraceutical compound than the parental mutualistic community of organisms.

The parental mutualistic community of organisms as used in the latter method is as defined for use in the former method presented herein.

In addition to the definition of the parental mutualistic community and its constituents as in the method presented further hereinabove, the method for generation of improved organisms or communities of organisms involves the cultivation of multiple parallel batches starting out from the same parental mutualistic community.

This cultivation may preferably be carried out for a time sufficient to be able to identify differences in characteristics, such as production of a nutrient or a nutraceutical compound, between the different parallel batches. In a preferred embodiment, the cultivation is carried out for at least ten days, more preferably for at least twenty days, even more preferably for at least one month, particularly preferably for at least two months, wherein serial transfers are carried out during this time as appropriate.

According to one preferred embodiment, the cultivation involves at least three, more preferably at least five, even more preferably at least ten, particularly preferably at least 15 serial transfers of the cultivated organisms/communities.

After appropriate cultivation, each parallel batch is analyzed and the nutrient-producing or nutraceutical compound-producing ability of each batch/community is determined.

For said determination, methods as are commonly known in the art may be used as long as they reflect the nutrient-producing or nutraceutical compound-producing ability with adequate accuracy or give a sufficient indication of said ability. In a preferred embodiment, the nutrient-producing ability or nutraceutical compound-producing ability is determined by analyzing the culture supernatant through a quantitative measurement of the nutrient or nutraceutical compound, more preferably the nutrient-producing ability or nutraceutical compound-producing ability is determined by analyzing culture supernatant samples using UPLC, HPLC, LC, colorimetric assays, fluorescence/spetcroscopic measurement, GC-MS, LC-MS, MS, enzymatic assays or biological assays, most preferably the nutrient-producing ability or nutraceutical compound-producing ability is determined by analyzing culture supernatant samples using Ultra Performance Liquid Chromatography (UPLC).

Preferably, the results of said determination are taken and compared with each other and/or the parental strain to identify the batches and/or communities which have evolved to achieve the best or highest nutrient-producing ability or nutraceutical compound-producing ability.

Further, the method for generation of improved organisms or communities of organisms involves a step of selecting a community of organisms which produces higher amounts of the nutrient or nutraceutical compound than the parental mutualistic community of organisms, preferably wherein the amount of the nutrient or nutraceutical compound produced by the parental mutualistic community has been determined before carrying out the step of cultivation.

Preferably, the method for the generation of improved organisms or communities of organisms further involves a step of isolating the improved nutrient-producing or nutraceutical compound-producing organism (preferably the second organism according to the definition herein) from the community after the step of selecting the highly producing community of organisms, more preferably wherein the improved nutrient-producing or nutraceutical compound-producing organism is then cultivated in monoculture.

According to one preferred embodiment, after having identified the improved nutrient-producing or nutraceutical compound-producing organism or community of organisms obtained by the method for the generation of improved organisms or communities of organisms, the improved organism or community of organisms is again subjected to the method or the generation of improved organisms or communities of organisms as described herein. Repeated rounds of directed mutation according to the method of the present invention is expected to lead to ever more improved strains having a desired property.

A nutrient-producing or nutraceutical compound-producing community of organisms as described in the methods of the present invention is also subject to the present invention and considered to exhibit unprecedented and desirable properties for producing said nutrient or nutraceutical compound and providing said compound to certain foods.

Using the method for the generation of improved organisms or communities of organisms according to the present invention, highly improved strains which are derived from natural isolates and have not been genetically modified can be obtained.

Thus, such highly desirable strains which can be obtained by the method for the generation of improved organisms or communities of organisms according to the present invention are also subject of the present invention.

All embodiments of the present invention as described herein are deemed to be combinable in any combination, unless the skilled person considers such a combination to not make any technical sense.

### Examples

There are references that demonstrate the ability of specific strains of Lactic acid bacteria (LAB) to produce and release beneficial compounds, like a number of B-vitamins, in their environment (LeBlanc et al.: "B-Group vitamin production by lactic acid bacteria - current knowledge and potential applications."; J Appl Microbial. 2011 Dec; 111(6): 1297-309.).

Strains available from natural isolates collections exhibited a high similarity on sequence level with the first gene (*ribG*) of the riboflavin operon from a known Riboflavin-producing Lactococcus lactis strain. However, vitamin production by LAB is considered a strain-dependent trait and just the existence of the operon is not sufficient evidence.

For this reason, the inventors reconstructed genome-scale metabolic models for two of the species, viz. *L. lactis* and *Brevibacterium casei.* The metabolic models predicted that these two strains contain all the necessary enzymes for the complete riboflavin production pathway and are also able to excrete the compound to their environment.

Finally, these strains were able to grow without the supplementation of riboflavin in the medium. Hence, they were selected as member organisms of a mutualistic community (second organism according to the definition of the present invention). The evolution niche was the chemically defined medium (CDM), used previously by the inventors to investigate interactions between *S*. *cerevisiae* and lactic acid bacteria (Ponomarova et al.: "Yeast Creates a Niche for Symbiotic Lactic Acid Bacteria through Nitrogen Overflow."; Cell Syst. 2017 Oct 25;5(4):345-357.).

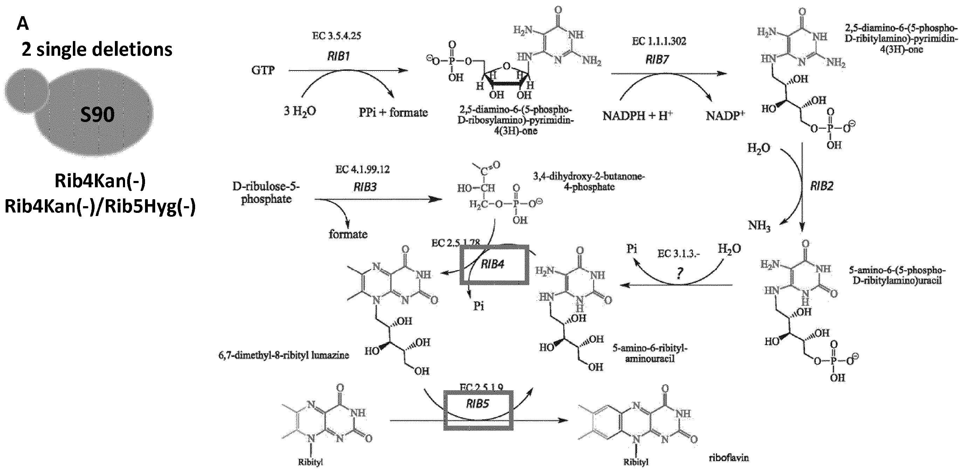

The other member (first organism according to the definition of the present invention) selected for the examples provided herein is from the *S*. *cerevisiae* species which is able to provide essential amino acids to the bacteria. However, yeasts are able to produce riboflavin on their own and therefore it was necessary to delete the synthetic pathway of riboflavin. We used the prototrophic yeast strain S90 and inserted deletions to create auxotrophic strains. A single mutant, lacking the Rib4 gene and a double mutant lacking both Rib4 and Rib5 genes (Figure 1A and 1B).

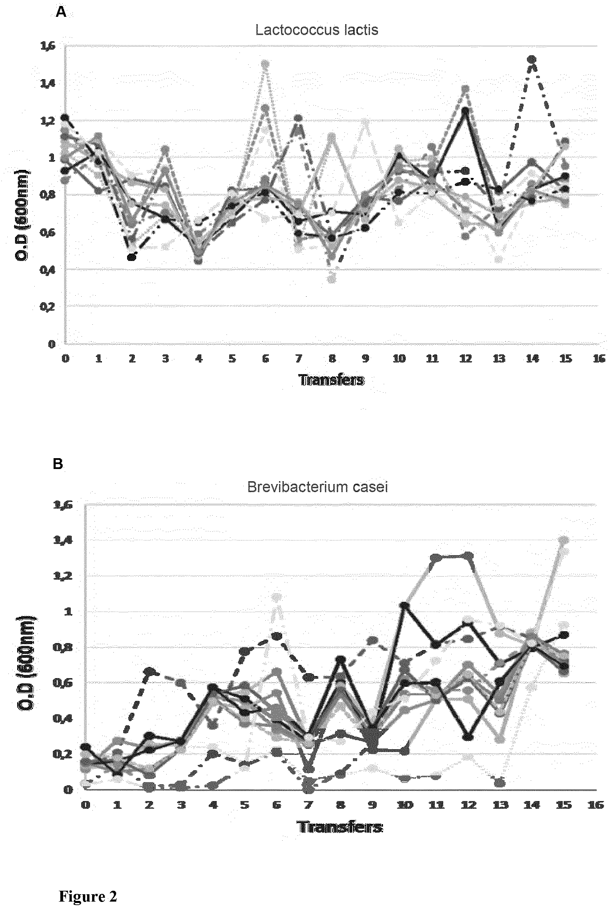

The communities that consisted of the single mutant yeast (Δ*rib4*) and one of the 2 bacterial species, respectively, were stable and so a serial transfer experiment started, with 12 initial populations for each community. The experiment lasted for two months (16 transfers), but since the populations are not monocultures, estimating the number of generations cannot be accurate (Figure 2).

After the completion of the serial transfer experiment and before the establishment of an analytics protocol in the UPLC for the detection of riboflavin, a way to screen isolates from the populations in a semi-high throughput way was needed. Riboflavin is a molecule that fluoresces at specific wave lengths, absorption 440nm, emission 520nm, a fact that can be used for the quantification of free riboflavin in the supernatant of monocultures.

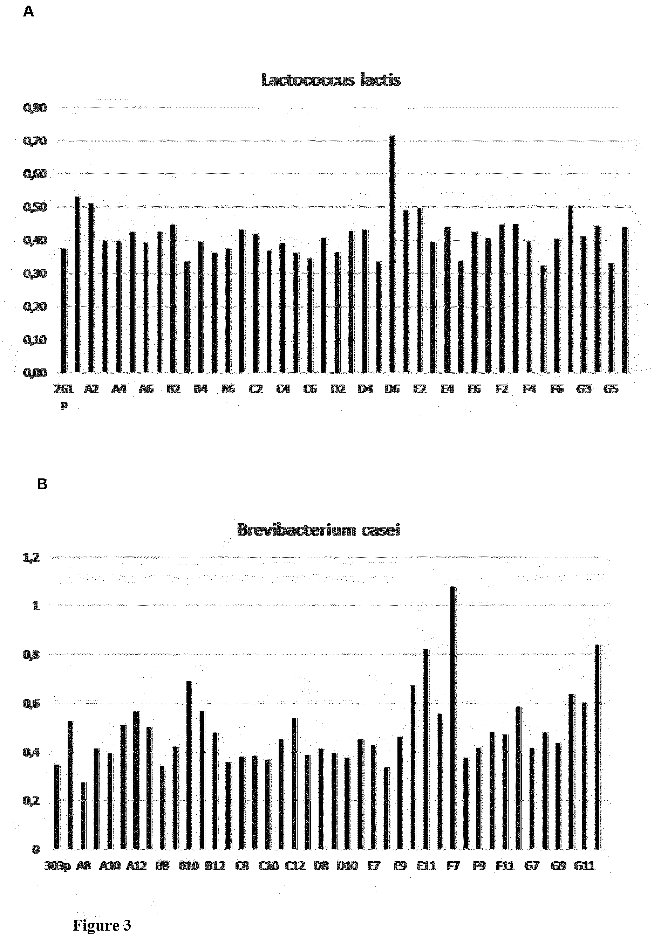

In total 84 strains of each species (*L. lactis* and *B*. *casei*) were selected and their producing abilities were assessed through absorbance measurement in a plate reader. The majority of the evolved isolates were able to produce higher amounts of riboflavin than their parental strains, with the best ones producing two to three times more riboflavin (Figure 3). From this assay, it appears that the two parental strains produce about the same amount of riboflavin and that the *L. lactis* isolates have been improved to produce around 0.5 µg/mL, while the *B*. *casei* isolates produce in general 0.4 µg/mL or more and the best ones between 0.7 and 0.9 µg/mL.

However, an analysis which is based on fluorescence alone should only be taken as a rough indication of the amounts of riboflavin produced, as the true values might be different due to other products of the bacterial metabolism which might affect the fluorescence measurement. For example, the biological active forms of riboflavin, FAD and FMN, absorb at similar wave lengths.

For higher precision, three evolved *L. lactis* strains and three evolved *B*. *casei* strains were selected for further phenotyping. In parallel a second round of evolution started with the final populations, aiming to achieve even higher production yields. The riboflavin producing ability of the parental and the evolved strains was followed over time throughout their growth curves and was calculated by analyzing supernatant samples with UPLC.

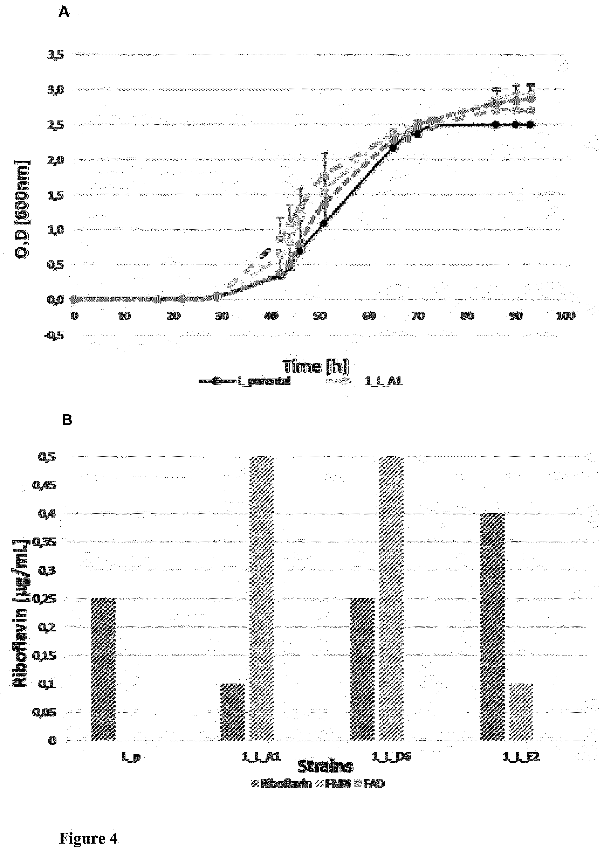

The growth curves were performed in the evolution medium which was supplemented with amino acids, as indicated by Ponomarova *et al*., 2017 (vide supra). Concerning the *L. lactis* isolates, the parental strain (L_p) is excreting into the environment around 0.3 µg/mL, while strain 1_L_E2 was the best one, releasing 0.5 µg/mL of riboflavin and around 0.2 µg/mL of FMN (Figure 4).

The maximum amount of free riboflavin in the supernatant was reached during the stationary phase, while one of the isolates, 1_L_A1, produced mostly FMN. The observed high production of FMN, might be associated with the fact that this molecule does not require any further transformation and can be used immediately by the yeast's metabolism.

The second round of evolution was carried out for the time of one month and the same procedure was followed for the isolation of single strains from the evolved populations. This time the results, based on fluorescence screening, were even more prominent.

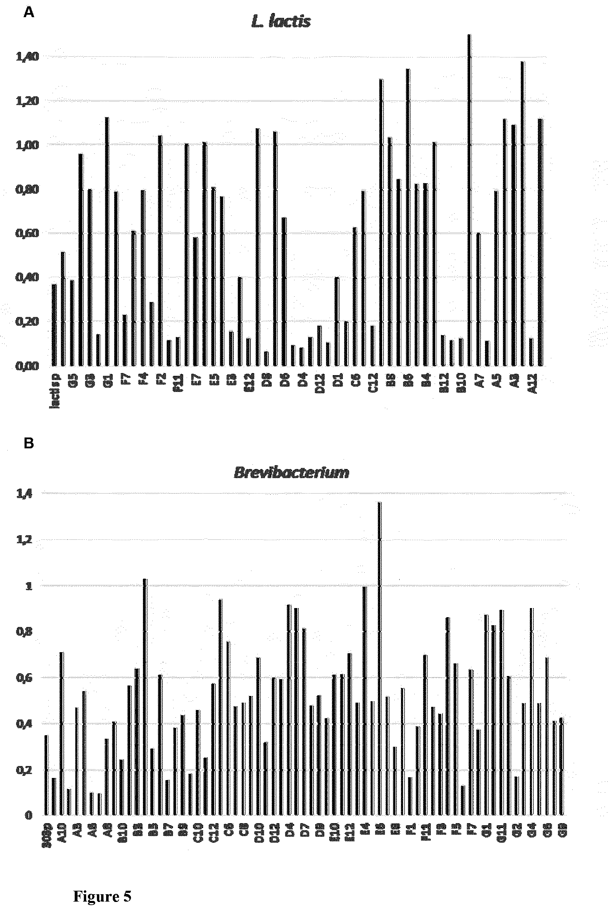

After the first round of evolution, none of the *L. lactis* isolates had been determined to produce amounts higher than 0.7 µg/mL, while this time the measured value for ten strains was ≥1 µg/mL. The general trend for the *B*. *casei* isolates was a two-fold further increase and two strains produced more than 1 µg/mL. On the other hand, it is interesting to note that on average one out of five isolates performed worse than the parental, a fact which might imply the possible rise of unfavorable mutations in the population (Figure 5). Thus, directed and targeted selection of favorable evolved organisms is key to the present invention.

From the second round of evolution, ten strains were selected in total for further phenotyping following the same approach as for the strains from the first round of evolution. The production of riboflavin was followed over time throughout their growth curves and was calculated by analyzing supernatant samples with UPLC.

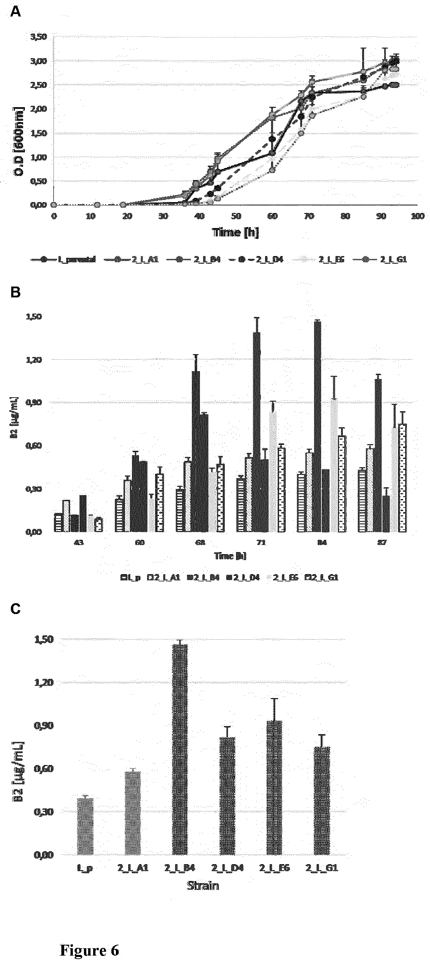

Similar to the first round of evolution the highest amounts of riboflavin are detected during the stationary phase and strain B4 was able to produce more than 1 µg/mL (Figure 6: a, c). What else can be observed is that during the growth curve, the levels of riboflavin are initially increasing, before they start decreasing, with most strains exhibiting similar production and consumption profiles.
However, it was not determined whether this is due to the fact that the strains take up riboflavin from the environment, or whether at this point most of the compound was converted to FMN and FAD (Figure 6b). Supernatants from specific time points were selected for LCMS analysis, in order to determine the levels of riboflavin, FMN and FAD with higher accuracy.

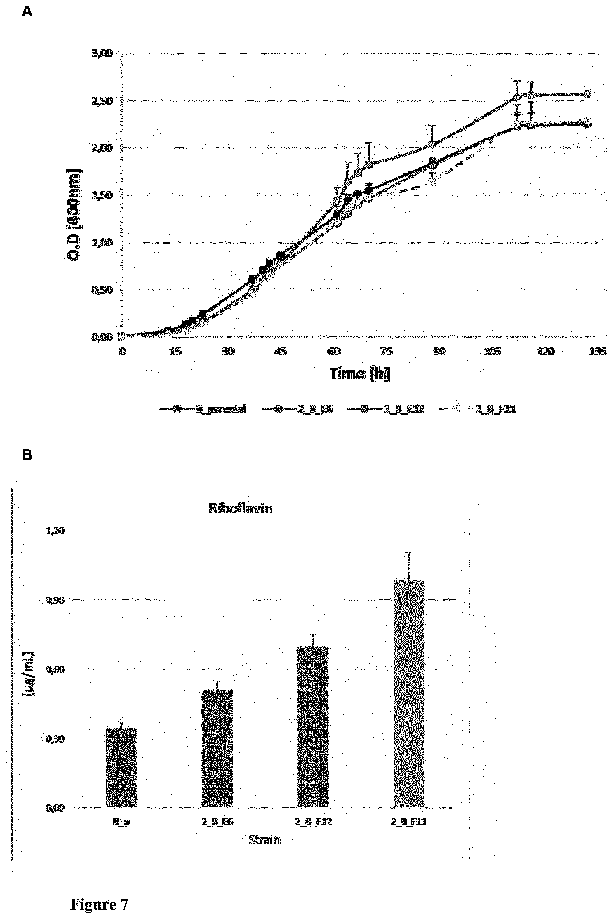

Similarly, the *B*. *casei* isolates' riboflavin yield was increased three-fold compared to their parental strain. Maximum riboflavin concentration was calculated at 1 µg/mL, during the stationary phase (Figure 7).

Part of this project was to connect higher production yields with better fitness, which can be translated as better growth in the evolution conditions. However, as it is clear from the growth curves shown in the previous section, the differences between the evolved strains and the parental are not so obvious both for growth rate and maximum OD.

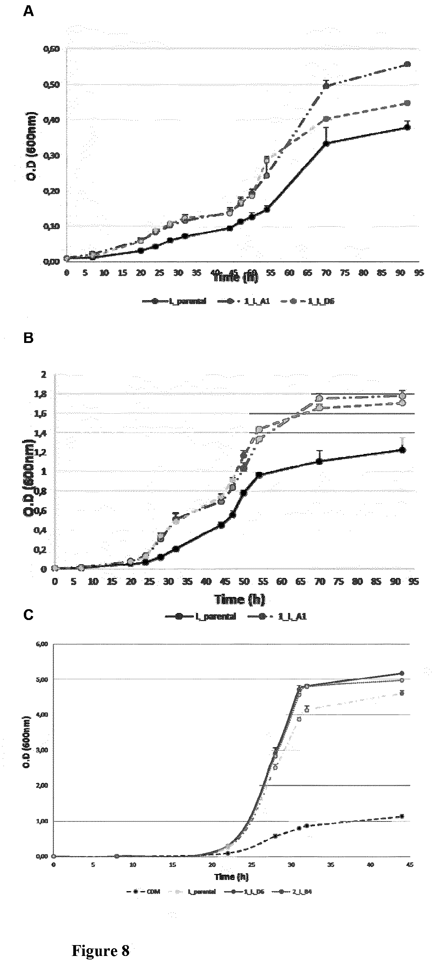

In order to study this phenomenon further, two different conditioned medium assays were performed. Firstly, wild type S90 was cultured in the evolution medium and the conditioned medium was collected at stationary phase. As a control for this assay, two times diluted amino acid supplemented CDM was used to simulate the depletion of nutrients during the growth of yeast cells. As can be observed in figure 7, the evolved strains performed better than their parental (Figure 8: a, b).

In the second part of the experiment conditioned medium from bacterial monocultures was collected at stationary phase and was diluted two times with fresh medium, in order to restore the amount of nutrients. The auxotrophic yeast mutant was cultured in the conditioned medium. Depletion of riboflavin and FMN from the conditioned medium was confirmed with UPLC at the end of the growth curve.

Normal growth of the yeast mutant cells was observed in all the spent media, independently if they were cultured in conditioned medium of the parental or the evolved strains. A slightly higher growth rate was observed for the mutant yeast cells cultured in conditioned medium originating from evolved strains (Figure 8c).

A possible explanation might be that the difference in riboflavin production between evolved and parental strains is not enough to lead to better growth of the yeast cells, or that the dilution of the nutrients doesn't favor differences in the growth pattern.

The example of riboflavin already confirms that directed evolution of complex metabolites with the use of a small community of selected organisms can be achieved.

In order to prove that it can be applied also for the production of different compounds and that different microbial species can be used, a similar community was formed for the production of folate (vitamin B9). The term folate is generally used to describe a group of compounds that derive from folic acid. These compounds differ by physicochemical characteristics, such as the state of oxidation and the number of glutamate residues.

The challenge in this case is the microbial metabolism of folate. The molecule contains one pterin moiety bound to para-aminobenzoic acid (pABA), which is produced from the pentose phosphate pathway. However, genome analysis of lactic acid bacteria showed that many strains that contained the genes for folate biosynthesis were not able to synthesize pABA and their phenotype was auxotrophic for folate.

Like in the case of riboflavin, *S*. *cerevisiae* is not dependent on environmental folate, as it is able to synthesize both pABA and folate. At the same time if the pathway for folate biosynthesis is disrupted, but pABA synthesis is undisrupted, then the cells can recycle other compounds like nucleotides to form folate.

For this reason, the approach selected was to introduce a deletion to ABZ1 gene (para-aminobenzoic synthase) and to modify the medium, by omitting both pABA and folate and by adding again riboflavin. Since the production of folate in bacteria requires also a second co-factor (DHPPP), a collection of strains was searched for the existence of strains that can potentially produce both. The genome scale model predicted that a specific strain of *Lactobacillus parakefiri* is able to produce folate without any supplementations in the medium.

This new community was evolved for two months and a total of 16 transfers. Lack of a way to screen strains on large scale for folate production, apart from microbiological assays, meant that only a small number of evolved strains can be isolated and the production is estimated with UPLC analysis. Preliminary results showed that one strain is able to produce 0.5 µg/mL and another one 0.1 µg/mL of a compound with the same retention time as the folic acid standard.

At the same time the parental strain did not produce this compound in detectable levels. Thus, evidence has been provided that the method of the present invention can equally be applied to other nutraceutical compounds and nutrients.

### Methods

### Reconstruction of genome-scale metabolic models

Reconstruction of genome-scale metabolic models was performed based on Machado *et al*., 2018 (Machado D et al., 2018; "Fast automated reconstruction of genome-scale metabolic models for microbial species and communities", Nucleic Acids Research, gky537, https://doi.org/10.1093/nar/gky537).

### Media and culture conditions

Prototrophic strains of *Saccharomyces cerevisiae* S90 and kefir isolates of lactic acid bacteria (*Lactococcus lactis, Brevibacterium casei* and *Lactobacillus parakefiri*) were pre-cultured overnight using YPAD and MRS respectively. The lactic acid bacteria were provided by Dr. S. Blasche (EMBL, Germany).

The chemically defined medium (CDM35) was described by Ponomarova *et al.,* 2017 (Ponomarova et al.: "Yeast Creates a Niche for Symbiotic Lactic Acid Bacteria through Nitrogen Overflow."; Cell Syst. 2017 Oct 25;5(4):345-357.) and was designed by reducing the number of components in the rich medium composed as a union of previously described media. Two different versions of the medium were used, in order to create the proper environment for the evolution. The first one is CDM35R-, from which riboflavin was removed and the other is CDM35Fol-PABA-, from which both folate and paraminobenzoic acid were removed.

Single bacteria cultures are supplemented with 40 mL/L of amino acids solution. Composition of this solution is according to Table 1 below:

**Table 1**

| Amino acid | Concentration (g/L) |
|---|---|
| L-Glutamic acid | 15 |
| L-Phenylalanine | 10 |
| L-Proline | 17.5 |
| L-Asparagine | 12.5 |
| L-Aspartic acid | 10.5 |
| L-Glutamine | 15 |
| L-Serine | 12.5 |
| L-Threonine | 12.5 |
| L-Cysteine | 5 |
| L-Alanine | 10 |
| Glycine | 7.5 |
| L-Lysine | 10 |
| L-Tryptophan | 5 |

Liquid medium experiments with single bacteria strains or yeast mutant strains were started at 0.01 OD₆₀₀ using pre-cultures washed 2 times with phosphate buffered saline (PBS). The washed yeast cells were starved for 3h prior to the start of the experiment. All cultures were grown statically at 30°C. To measure growth rate and vitamin production of single strains CDM35 was supplemented with 40 mL/L of amino acids solution and cultures were grown statically in 50mL erlenmeyer flasks. OD₆₀₀ measurements were made using a spectrophotometer.

### Adaptive laboratory evolution

Three different mutualistic communities were constructed. One consisted of *S*. *cerevisiae* and *L. lactis,* the second of *S*. *cerevisiae* and *B*. *casei* and the third of *S*. *cerevisiae* and *L. parakefiri.* Multiple parallel co-cultures of the two communities were started, with each containing both members in 1:1 ratio (OD600). Initial OD600 of the communities was 0.01 and were grown in 96 well plates containing 200 µL CDM.

The communities were grown microaerobically (statically) at 37 °C. Growth was monitored in a Tecan microplate reader and when the communities reached stationary phase they were transferred to fresh medium with an OD of 0.01.

Stability of the communities was estimated through plating on YPAD plates, where all members could grow and form colonies. Intermediate and final populations were stored by adding 80% w/v glycerol directly to the plate wells (final concentration 30% w/v) and then storing it at -80°C.

### Concentration measurements

Single bacterial strains (parental and evolved) were cultured as described above and supernatant samples were collected at regular intervals. The samples were filtered with 0.22 µm syringe filters and were injected to the UPLC machine and the protocol proposed by Waters Corporation (Aubin and Hynes, 2006; Application NOTE from Waters Corporation, Milford, MA, USA on "UPLC™/PDA/MS Analysis of Riboflavin and related compounds") was generally followed.

### Conditioned media assays

### Bacterial conditioned medium

Bacterial strains were cultured in CDM35R- medium for 72h and then the medium was filtered with 0.22 µm syringe filters. Filtered conditioned medium was then mixed 1:1 with fresh CDM35R- to replace the used-up nutrients. Auxotrophic yeast cells were inoculated with an initial OD600 of 0.01 in 50 mL erlenmeyer flasks, which contained 7 mL of the conditioned/fresh CDM35R- mixture. At the end of the yeast growth, supernatant samples were collected and the concentration of the desired compounds was determined with UPLC, as described above.

### Yeast conditioned medium

Wild type *S*. *cerevisiae* S90 cells were cultured in CDM35R-, until they reached mid-exponential phase. Then the medium was filtered with 0.22 µm syringe filters and bacterial cells (parental and evolved) were inoculated with initial OD600 of 0.01. The inoculation was done in 50 mL erlenmeyer flasks, which contained 12 mL of the yeast spent medium. Supernatant samples were collected during the different growth phases for nutrient measurement, with use of UPLC as described above.

### Diluted CDM35R- medium

In order to study the differences in growth when different amounts of amino acids are supplemented to single bacterial strains, we diluted 1:1 CDM35R-, supplemented with amino acids, with sterile ddH₂O. Bacterial cells (parental and evolved) were inoculated with an initial OD600 of 0.01 in 50 mL erlenmeyer flasks, with 12 mL of the diluted CDM35R-. Supernatant samples were collected during the different growth phases for nutrient measurement, with use of UPLC as described above.

## Claims

1. Method for the production of a nutrient or a nutraceutical compound comprising:
a) provision of a parental mutualistic community of organisms comprising:
I. a first organism which is able to produce and excrete into the environment a first set of compounds and which requires said nutrient or nutraceutical compound to be produced for optimal proliferation, and
II. a second organism which is able to produce and excrete into the environment said nutrient or nutraceutical compound, and which requires said first set of compounds for optimal proliferation,
b) cultivation of the parental mutualistic community of organisms in a culture comprising a growth medium which is devoid of or contains levels of the nutrient or nutraceutical compound and of the set of compounds mentioned in step a) I. above which are insufficient to allow optimal proliferation of the first and the second organisms,
c) harvesting the nutrient or nutraceutical compound from the culture medium.

2. Method according to claim 1, wherein the first organism is a microbial strain and/or wherein the second organism is a microbial strain.

3. Method according to claim 1 or 2, wherein the first organism is from the phylum *Ascomycota,* preferably from the class *Saccharomycetes,* more preferably from the genus *Saccharomyces,* particularly preferably of the species *Saccharomyces cerevisiae* and/or wherein the first organism is auxotrophic for the nutrient or nutraceutical compound to be produced.

4. Method according to claim 3, wherein the auxotrophy is due to functional impairment of a gene, preferably due to gene deletion, more preferably due to deletion of genes in the synthetic pathway for production of the nutrient or nutraceutical compound by the first organism.

5. Method according to any of claims 1 to 4, wherein the first set of compounds comprises amino acids, preferably wherein the first set of compounds comprises amino acids glutamine and serine, more preferably wherein the first set of compounds consists of amino acids glutamine and serine.

6. Method according to any of claims 1 to 5 wherein the second organism is from the domain *Bacteria,* preferably from the group of lactic acid bacteria (LAB), more preferably of the species *Lactococcus lactis, Lactobacillus parakefiri* or *Brevibacterium casei* and/or wherein the second organism is auxotrophic for the first set of compounds, preferably wherein the second organism is auxotrophic for one or more amino acids, more preferably wherein the second organism is auxotrophic for glutamine and/or serine, and/or wherein the second organism is a natural isolate and/or wherein the second organism is not a genetically modified organism (GMO).

7. Method according to any of claims 1 to 6, wherein the nutrient or nutraceutical compound is a vitamin, preferably wherein the nutrient or nutraceutical compound is a B vitamin, more preferably wherein the nutrient or nutraceutical compound is Vitamin B2 (riboflavin) or Vitamin B9 (folate).

8. Method according to any of claims 1 to 7, wherein the growth medium does not comprise amino acids, in particular serine and glutamine, in levels sufficient to allow optimal proliferation of the first and second organisms, alternatively wherein the growth medium does not comprise the nutrient or nutraceutical compound to be produced in levels sufficient to allow optimal proliferation of the first and second organisms, more preferably wherein the growth medium does not comprise any of serine, glutamine or the nutrient or nutraceutical compound to be produced in levels sufficient to allow optimal proliferation of the first and second organisms, most preferably wherein the growth medium does not comprise any of serine, glutamine, and Vitamin B2 or B9 in levels sufficient to allow optimal proliferation of the first and second organisms.

9. Method for the generation of an improved nutrient-producing or nutraceutical compound-producing organism or community of organisms comprising:
a) cultivation of a parental mutualistic community of organisms as defined in any of claims 1 to 8 in one or multiple parallel batches,
b) determination of the nutrient-producing or nutraceutical compound-producing ability of each community and/or each batch after cultivation,
c) selection of a community of organisms which produces higher amounts of the nutrient or nutraceutical compound than the parental mutualistic community of organisms.

10. Method according to claim 9, wherein the nutrient-producing or nutraceutical compound-producing organism is subsequently isolated from the community of organisms selected in step c., preferably wherein the nutrient-producing or nutraceutical compound-producing organism is then propagated in monoculture.

11. Method according to claim 9 or 10, wherein the cultivation comprises serial transfers of the cultivated communities, preferably wherein the cultivation comprises at least 3, more preferably at least 5, even more preferably at least ten, most preferably at least 15 serial transfers of the cultivated communities.

12. Method according to any of claims 9 to 11, wherein the nutrient-producing ability or nutraceutical compound-producing ability is determined by measuring the amount of nutrient or nutraceutical compound present in the medium, preferably wherein the nutrient-producing ability or nutraceutical compound-producing ability is determined by analyzing the culture supernatant through a quantitative measurement of the nutrient or nutraceutical compound, more preferably wherein the nutrient-producing ability or nutraceutical compound-producing ability is determined by analyzing culture supernatant samples using UPLC, HPLC, LC, colorimetric assays, fluorescence/spetcroscopic measurement, GC-MS, LC-MS, MS, enzymatic assays or biological assays, most preferably wherein the nutrient-producing ability or nutraceutical compound-producing ability is determined by analyzing culture supernatant samples using Ultra Performance Liquid Chromatography (UPLC).

13. Method according to any of claims 9 to 12, wherein the organisms selected for producing high amounts of the nutrient or nutraceutical compound are repeatedly subjected to steps I. to III. as defined in any of claims 9 to 12.

14. Nutrient-producing or nutraceutical compound-producing community of organisms as defined in any of claims 1 to 8.

15. Nutrient-producing or nutraceutical compound-producing organism obtained by the method of any of claims 9 to 13.
